Europäisches Patentamt

European Patent Office

Office européen des brevets

(11) Publication number: **0 419 740 A1**

# EUROPEAN PATENT APPLICATION

(21) Application number: 89309828.5

(51) Int. Cl.5: **A61F 2/16**

(22) Date of filing: **27.09.89**

(43) Date of publication of application:
**03.04.91 Bulletin 91/14**

(84) Designated Contracting States:
**AT BE CH DE ES FR GB GR IT LI LU NL SE**

(71) Applicant: **HANITA LENSES (a Limited Partnership)**

**Kibbutz Hanita 22885(IL)**

(72) Inventor: **Porat, Menachem**

**Kibbutz Hanita 22885(IL)**

(74) Representative: **Goodanew, Martin Eric et al
MATHISEN, MACARA & CO. The Coach
House 6-8 Swakeleys Road
Ickenham Uxbridge UB10 8BZ(GB)**

(54) **Negative-power intraocular lens.**

(57) A negative-power intraocular lens, having a central, active zone (CZ) and an annular, marginal zone (MZ), the diameter ($D_1$) of which central zone (CZ) is substantially smaller than the outside diameter ($D_2$) of the lens, the thickness of the lens increasing from the center thereof towards the periphery of the central zone (CZ) and decreasing from the periphery across the marginal zone (MZ) towards the edge of the lens.

*Fig. 1.*

# NEGATIVE-POWER INTRAOCULAR LENS

The present invention relates to a negative-power intraocular lens, that is, a negative-power lens to be surgically implanted as a substitute for the human crystalline lens.

Patients afflicted by macular degeneration, or other retinal conditions that mainly affect central vision, may suffer a serious decrease of their visual acuity to the point where they are no longer able to recognize fine patterns, which in fact means that they are losing their capacity to read and write or to drive cars. As there is no effective treatment once significant symptoms have appeared, patients are reduced to relying on optical aids that, so far, are often ineffectual and always difficult to use. For distance vision, various miniature, so-called low-vision telescopes have been proposed, that are mounted on spectacle frames. These devices, however, drastically reduce the patient's field of view from the normal 150° (in the horizontal meridian) and 120° (in the vertical meridian) to as low as 26° for a magnification of 1.7x and 9-11° for a magnification of 3x. Moreover, these telescopes are awkward, difficult to fit, and not very appealing cosmetically. Due to these drawbacks, these telescopes are used by very few patients only. For near vision, many patients use hand-held magnifiers, which demand both coordination and steadiness of hand, are tiring for elderly people, and provide only a limited field.

It has been suggested to use a Galilean telescope system consisting of a positive spectacle lens and a high-power negative contact lens. However, high-power negative contact lenses are relatively heavy and are difficult to maintain in position relative to the spectacle lens. These systems were therefore found impractical.

Encouraged by the present state of art of surgical techniques for substituting plastic intraocular lenses (IOLs) for human cataractous crystalline lenses, attempts were made to utilize IOLs of negative power to modify the optical properties of the eye so as to produce significant retinal magnification while avoiding the undesirable loss of visual field associated with low-vision telescopes. This was achieved by replacing the crystalline lens with a strongly negative IOL which, when combined with a strongly positive spectacle lens, turns the eye into an integral part of an ocular telephoto system, providing a substantially greater field of vision at a significant magnification.

While the potential usefulness of this arrangement has been fully established, difficulties were encountered due to the fact that with a negative lens, the thickest part is at the periphery. The heavy rim of these lenses, it was found, interfered with the actual implanting, the positioning and maintaining the position, of the IOL, and endangered the delicate tissues in which it is embedded.

It is one object of the present invention to overcome the drawbacks of the prior art IOLs and to provide a high negative-power IOL that has a rim configuration substantially comparable to that of the conventional positive power IOLs.

This the invention achieves by providing a negative-power intraocular lens, having a central, active zone and an annular, marginal zone, the diameter of which central zone is substantially smaller than the outside diameter of said lens, the thickness of said lens increasing from the center thereof towards the periphery of said central zone and decreasing from said periphery across said marginal zone towards the edge of said lens, the active portion of one surface of said lens being a surface of revolution generated by a generatrix constituted by a monotonic curve.

The invention will now be described in connection with certain preferred embodiments with reference to the following illustrative figures so that it may be more fully understood. With specific reference now to the figures in detail, it is stressed that the particulars shown are by way of example and for purposes of illustrative discussion of the preferred embodiments of the present invention only and are presented in the cause of providing what is believed to be the most useful and readily understood description of the principles and conceptual aspects of the invention. No attempt is made to show structural details of the invention in more detail than is necessary for a fundamental understanding of the invention, the description taken with the drawings making apparent to those skilled in the art how the several forms of the invention may be embodied in practice.

In the drawings:

Fig. 1 is a cross-sectional view of a first, biconcave embodiment of the IOL according to the invention.

Fig. 2 is a similar view of a second, plano concave embodiment of the IOL, and

Fig. 3 represents a third, concavo-convex embodiment of the IOL according to the invention.

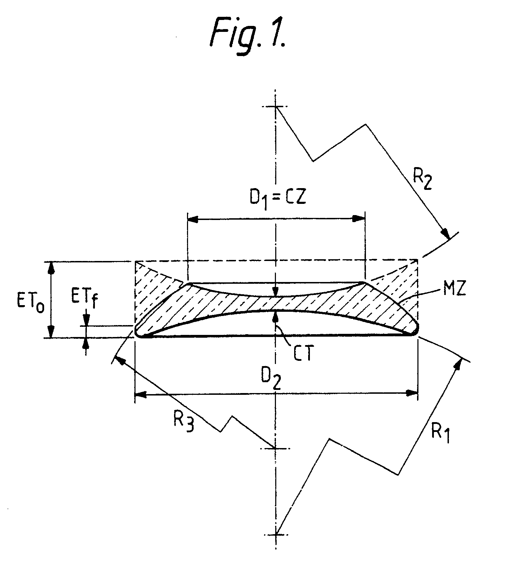

Referring now to the drawings, there is shown in Fig. 1 a negative, biconcave intraocular lens (IOL) according to the invention. It is seen that the lens consists of a central zone (CZ), which is the active zone and is delimited by the diameter $D_1$, which is substantially smaller than the outside diameter $D_2$ of the lens, and of a peripheral or marginal zone (MZ) which extends from the central zone outwards. The lens portion that was machined away to produce the marginal zone (MZ) is shown in dashed lines and the effect of this zone on the final edge thickness $ET_f$ is clearly seen when the latter is compared to the original edge thickness $ET_o$. In this embodiment, the surface constituting the marginal zone (MZ) is spherically convex. Also marked are the central thickness CT which is typically about 0.2 mm and the edge thickness - about 0.4 mm. To obtain a lens of a negative power of -40D (dioptres), the radius of curvature $R_1$ of the posterior surface would be about 7.7 mm and the radius of curvature $R_2$ of the central zone of the anterior surface, about 7.9 mm. To obtain a central zone of a diameter $D_1$ = 4 mm, at an outside diameter $D_2$ = 6 mm and an edge thickness of 0.4 mm, the radius of curvature $R_3$ of the marginal zone (MZ) would have to be $R_3$ = 4 mm. The central-zone diameter $D_1$ is envisaged to vary between 2 and 5 mm, and the outside diameter $D_2$ between 6 and 7 mm. Assuming $ET_f$ and CT to be substantially equal for all variants of the IOL of Fig. 1, $R_3$ would have to be varied to obtain the desired central-zone diameter $D_1$.

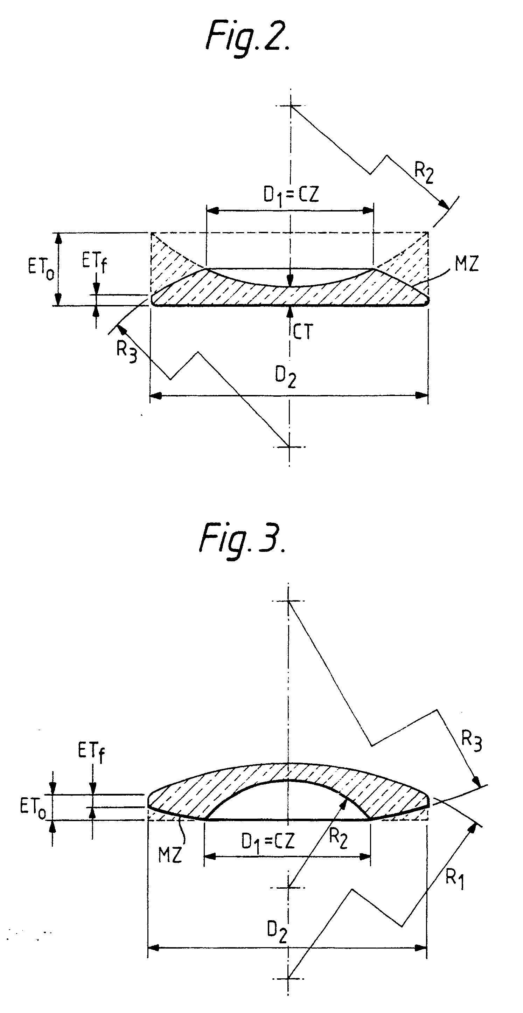

Fig. 2 shows a plano-concave embodiment of the IOL according to the invention. While notation is the same as in Fig. 1 and $ET_f$ as well as $D_1$ are also within the values given for the embodiment of Fig. 1, it is obvious that, for the same power, $R_2$, the radius of curvature of the concavity of the central zone CZ must be smaller than the corresponding $R_2$ of Fig. 1.

Similar considerations apply also to the embodiment shown in Fig. 3, which is a concavo-convex or meniscus type.

As mentioned in the introduction, the IOLs of the present invention are intended to be used in conjunction with high-power positive spectacle lenses.

While the surface of the marginal zone MZ was so far shown as spherically convex, it could in fact be spherically concave, aspherical, conical or toroidal and, having in general no optical function, it could even be rendered opaque. Yet, the above notwithstanding, cases are envisaged in which this zone may fulfill such a function, e.g., when the user removes his or her spectacles. Certain patients may profit from such an arrangement, in which case the exact curvature of the marginal zone would, of course, be of importance.

It should be noted that either one or both of the optically active surfaces could also be aspherical.

No reference was made in the above of the mounting braces or manipulating holes which could be the same type as used with positive cataract IOLs.

The IOL according to the invention can be made of any of the materials used for conventional IOL's or contact lenses

Apart from its above-explained function in reducing the thickness of the lens edge $ET_f$, the marginal zone MF is envisaged to serve a further purpose: By configuring it, in conjunction with the corresponding portion of the opposite surface, as an annular portion of a positive lens, a distinct improvement of distance vision is likely to be achieved with certain patients also without the above-mentioned positive spectacle lenses. Such a positive annulus would have a power of between + 0.25 and + 30 D.

It will be evident to those skilled in the art that the invention is not limited to the details of the foregoing illustrative embodiments and that the present invention may be embodied in other specific forms without departing from the spirit or essential attributes thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

## Claims

1. A negative-power intraocular lens, having a central, active zone and an annular, marginal zone, the diameter of which central zone is substantially smaller than the outside diameter of said lens, the thickness of said lens increasing from the center thereof towards the periphery of said central zone and decreasing from said periphery across said marginal zone towards the edge of said lens, the active portion of one surface of said lens being a surface of revolution generated by a generatrix constituted by a monotonic curve.

2. The intraocular lens as claimed in claim 1, wherein the power of said lens may vary over a range of -0.25 to -100 diopters.

3. The intraocular lens as claimed in claim 1, wherein the diameter of the central zone may vary over a range of 2 to 5 mm.

4. The intraocular lens as claimed in claim 1, wherein said lens is of a generally biconcave configuration.

5. The intraocular lens as claimed in claim 1, wherein said lens is of a generally plano-concave configuration.

6. The intraocular lens as claimed in claim 1, wherein said lens is of a generally concavo-convex configuration.

7. The intraocular lens as claimed in claim 1, wherein said marginal zone is configured, in conjunction with the corresponding portion of the opposite surface, to constitute an annular portion of a positive lens of a power of between +0.25 and +30 D.

# Fig.1.

# Fig.2.

# Fig.3.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 906 115 (ALLERGAN) <br> * Page 7, line 6 - page 8, line 10; page 14, line 8 - page 15, line 12; figures 1,6 * | 1,4 | A 61 F 2/16 |
| Y | | 3,6 | |
| A | | 2,7 | |
| | --- | | |
| Y | WO-A-8 301 566 (KOESTER) <br> * Abstract; page 7, lines 15-18; page 8, lines 11,12; page 10, lines 9-11; claims 1,4,11; figures 1-3 * | 3 | |
| A | | 1,2 | |
| | --- | | |
| Y | EP-A-0 242 043 (ALLERGAN) <br> * Column 3, lines 25-27,35-37; figures 1,2c,2e,2g * | 6 | |
| A | | 4 | |
| | --- | | |
| A | US-A-4 710 193 (VOLK) <br> * Column 19, lines 60-64; figure 8d * | 5 | |
| | --- | | |
| A | US-A-4 666 446 (KOZIOL) | | TECHNICAL FIELDS SEARCHED (Int. Cl.5) |
| | --- | | |
| A | WO-A-8 603 961 (VANNAS) | | A 61 F |
| | --- | | |
| A | WO-A-8 707 496 (PRECISION-COSMET) | | |
| | ----- | | |

The present search report has been drawn up for all claims

| Place of search | Date of completion of the search | Examiner |
|---|---|---|
| THE HAGUE | 23-05-1990 | KLEIN C. |